# EUROPEAN PATENT APPLICATION

(11) **EP 2 319 486 A1**
(43) Date of publication of application: **11.05.2011**
(21) Application number: 11154928.3
(22) Date of filing: 23.01.2009
(51) Int. Cl.: A61K 8/27, A61K 8/81, A61K 8/37, A61Q 17/04

(54) **Enhanced photoprotective compositions**

(30) Priority: 23.01.2008 US 22940 P
(62) Divisional of application: 09703295.7
(71) Applicant: Playtex Products, INC., Shelton, CT 06484 (US)
(72) Inventor: Spaulding, Laura A., Wayne, NJ 07470 (US); Pattillo, A. Christopher, Lake Hopatcong, CT 07849 (US)
(74) Representative: Neelissen-Subnel, Marianne

(57) **Abstract**

A photoprotective composition including a synergistic triplet combination of zinc oxide, styrene/acrylate copolymer and glycol. As a result of the inclusion of the synergistic triplet combination in a photoprotective composition, an unexpected boost in SPF is observed, as compared to a composition without the synergistic triplet combination.

## Description

### Cross Reference to Related Application

This application claims the benefits of U.S. Provisional Patent Application Serial No. 61/022,940, filed January 23, 2008, entitled "Enhanced Photoprotective Compositions," the content of which is incorporated herein by reference in its entirety.

### Technical Field

The present invention is generally directed to photoprotective compositions and, more particularly, to photoprotective compositions in the form of topically applied sunscreens that provide protection from ultraviolet (UV) radiation.

### Background

The ability of a photoprotective composition to provide protection against UV radiation is typically expressed as the sun protection factor (SPF) of the composition. Photoprotective compositions having SPF values of 80 or more generally contain 15% homosalate, which is an acceptable amount of homosalate for compositions in the U.S. market. However, compositions in the international market are allowed to include only 10% homosalate. To maintain an SPF of 80 or more for the products available in the international market, it is therefore desirable to limit the homosalate thereof to 10%.

Zinc oxide has been indicated as being a material that is inherently pyroelectric. Pyroelectricity is the ability of a certain material to generate an electrical potential when the material is heated or cooled. As a result of this change in temperature, positive and negative charges migrate to opposite ends of the zinc oxide lattice structure (the material becomes polarized). Thus, an electrical potential is established.

Zinc oxide is also a semiconductor with a direct band gap energy (Eg) of 3.37 eV at room temperature. Most zinc oxide has n-type character (as opposed to p-type character, which is more difficult to attain), which means that electron energy levels near the top of the band gap allow an electron to be excited into the conduction band with relative ease. Native defects such as oxygen vacancies or zinc interstitials are often assumed to be the origin of this n-type character. Intentional doping of the n-type zinc oxide, which may be effected by introducing aluminum, indium, or excess zinc into the zinc oxide structure, allows the zinc oxide to be formed into thin films in which the zinc oxide serves as a transparent conducting oxide, which can be used to form a transparent electrode.

When a photon hits zinc oxide, one of three things can happen: (1) the photon can pass straight through the zinc oxide, which happens for lower energy photons; (2) the photon can reflect off the surface; or (3) the photon can be absorbed by the zinc oxide. If the photon is absorbed by the zinc oxide, either heat or electron-hole pairs may be generated. Electron-hole pairs are generated if the photon energy is higher than the zinc oxide band gap value.

An incident photon may be absorbed by the zinc oxide if its energy is greater than the semiconductor band gap energy. As a result, an electron from the valence band of the zinc oxide (wherein the electron comes from the oxygen) is promoted into the conduction band (the metal ion orbital). If the energy of the incident photon is less than the band gap energy, it will not be absorbed as this would require that the electron be promoted to within the band gap. This energy state is forbidden. Once promoted into the conduction band, however, the electron relaxes to the bottom of the conduction band with the excess energy emitted as heat to the crystal lattice.

When a photon is absorbed, its energy is given to an electron in the crystal lattice. Usually this electron is in the valence band and is tightly bound in covalent bonds between neighboring atoms, and hence unable to move far. The energy given to it by the photon "excites" the electron into the conduction band where it is free to move around within the semiconductor. The covalent bond that the electron was previously a part of now has one fewer electron (which results in the formation of a "hole"). The presence of a missing covalent bond allows the bonded electrons of neighboring atoms to move into the hole, thereby leaving another hole behind, and in this way a hole can "move" through the lattice. Thus, it can be said that photons absorbed in the semiconductor create mobile electron/hole pairs.

The holes act as positive particles in the valence band. Both the electrons and the holes are free to migrate around the zinc oxide particle. Electrons and holes may recombine emitting photons of energy equal to the band gap energy. However, the lifetime of an electron/hole pair is quite long due to the specific nature of the electronic band structure. Thus, there is sufficient time for an electron and a hole to migrate to the surface and react with absorbed species.

A photon need only have a greater energy than that of the band gap in order to excite an electron from the valence band into the conduction band. In the solar frequency spectrum, much of the radiation reaching the surface of the earth is composed of photons with energies greater than the band gap of silicon (1.12 eV) and zinc oxide (3.37 eV). The higher energy photons will be absorbed by the difference in energy between these photons, and the band gap energy is converted into heat via lattice vibrations (called phonons).

### Summary of the Present Invention

The present invention differs from other topically applied photoprotective compositions due to the unexpected synergistic effect of a triplet combination of zinc oxide, styrene/acrylates copolymer and glycol, which result in a more inherent UV absorption activity that boosts SPF as compared to the activity of conventional zinc oxides alone. The use of low levels (1-5%) of zinc oxide in the triplet combination of components produce a more than anticipated increase in SPF.

The present invention can be used in conjunction with lower SPF products as well and in combination with other internationally approved sunscreen materials (i.e., photoprotective compositions for the international market in which the homosalate content is limited to 10%).

An unexpected, larger boost in in-vitro SPF was observed in photoprotective formulations containing the triplet combination of components according to the present invention (in-vitro SPF was unexpectedly increased by about 30 units), as compared to those compositions without the triplet combination. While not wishing to be bound by any particular theory, this increase in SPF is theorized to be the result of not only the zinc oxide behaving as a physical blocker, but also the result of the zinc oxide behaving as a UV absorber with significant absorbing ability for such a small amount of material present in the thin film formulation, as a result of the synergistic triplet combination according to the present invention.

### Brief Description of the Drawing

FIG. 1 is a graphical representation illustrating the photostability of a sunscreen composition having no zinc oxide, no styrene/acrylate copolymer, no caprylyl glycol, and 10% homosalate.
FIG. 2 is a graphical representation illustrating the photostability of a sunscreen composition having no zinc oxide, 5% styrene/acrylate copolymer, no caprylyl glycol, and 10% homosalate.
FIG. 3 is a graphical representation illustrating the photostability of a sunscreen composition having 3% zinc oxide, 5% styrene/acrylate copolymer, no caprylyl glycol, and 10% homosalate.
FIG. 4 is a graphical representation illustrating the photostability of a sunscreen composition having 3% uncoated zinc oxide, 5% styrene/acrylate copolymer, 1% caprylyl glycol, and 10% homosalate.
FIG. 5 is a graphical representation illustrating the photostability of a sunscreen composition having 2.5% uncoated zinc oxide, 5% styrene/acrylate copolymer, 1% caprylyl glycol, and 10% homosalate.
FIG. 6 is a graphical representation illustrating the photostability of a sunscreen composition having 3% uncoated zinc oxide, no styrene/acrylate copolymer, no caprylyl glycol, and 10% homosalate.
FIG. 7 is a graphical representation illustrating the photostability of a sunscreen composition having no zinc oxide, no styrene/acrylate copolymer, 1% caprylyl glycol, and 10% homosalate.
FIG. 8 is a graphical representation illustrating the photostability of a sunscreen composition having 2.5% uncoated zinc oxide, 5% styrene/acrylate copolymer, 1% caprylyl glycol, and 10% homosalate.
FIG. 9 is a graphical representation illustrating the photostability of a sunscreen composition having 2.5% coated zinc oxide, 5% styrene/acrylate copolymer, 1% caprylyl glycol, and 10% homosalate.
FIG. 10 is a graphical representation illustrating the photostability of a sunscreen composition having 2.5% coated zinc oxide, 5% styrene/acrylate copolymer, 1% caprylyl glycol, and 10% homosalate.
FIG. 11 is a graphical representation illustrating the photostability of a sunscreen composition having 5% (2.5% active) dispersed zinc oxide, 5% styrene/acrylate copolymer, 1% caprylyl glycol, and 10% homosalate.
FIG. 12 is a graphical representation illustrating the photostability of a second sunscreen composition having 5% (2.5% active) dispersed zinc oxide, 5% styrene/acrylate copolymer, 1% caprylyl glycol, and 10% homosalate.
FIG. 13 is a graphical representation illustrating the photostability of a sunscreen composition having 3% uncoated zinc oxide, 5% styrene/acrylate copolymer, 1% diethylhexyl 2,6-naphthalate, and 10% homosalate.

### Description of the Preferred Embodiments

All percentages of components are weight percentages.

One component of the synergistic triplet combination according to the present invention is zinc oxide. The zinc oxide may be coated, uncoated, in a dispersion or any combinations thereof. The zinc oxide is present in a photoprotective composition in an amount about 1% to about 15%, based on the total weight of the composition. In one embodiment, the zinc oxide is present in an amount about 1% to about 5%, based on the total weight of the composition. In another embodiment, the zinc oxide is present in an amount about 2% to about 3%, based on the total weight of the composition.

In one photoprotective composition according to the present invention, 5% of zinc oxide was added as a component to reduce a homosalate concentration from 15% to 10% (e.g., about 9% to about 11%). This zinc oxide component contains approximately 50% zinc oxide, which means approximately 2.5% of active zinc oxide is added to the composition via the zinc oxide component. In another embodiment according to the present invention, a zinc oxide component is used in combination with hollow styrene/acrylate copolymer spheres that are manufactured via an emulsion polymerization process. In yet another embodiment of the present invention, the zinc oxide and styrene/acrylate copolymer is used with a glycol, forming a triplet combination of components that results in a synergistic effect and unexpected boost in SPF as compared to a photoprotective composition without the triplet combination of the present invention.

The zinc oxide component of the present invention and for use in the photoprotective composition of the present invention can have an average particle size of up to about 2.74 microns and still be transparent on the skin after application of a formulated product that includes this zinc oxide component. One zinc oxide that may be of particular use with regard to the present invention is a formulation that includes 40-60% zinc oxide, 30-59% C₁₂₋₁₅ alkyl benzoate, and 1-5% isostearic acid (commercially available as ZinClear-IM 50AB from Advanced Nanotechnology). The present invention is not limited to the use of C₁₂₋₁₅ alkyl benzoate, however, as other esters are within the scope of the present invention. Other zinc oxide components that may be used include, but are not limited to, 40-60% zinc oxide, 30-59% caprylic/capric triglyceride, and 1-5% glyceryl isostearate (commercially available as ZinClear-IM 50CCT); uncoated zinc oxide (commercially available as Zano 10 from Umicore Zinc Chemicals); coated zinc oxide having an average particle size diameter of about 30-50 nanometers (commercially available as Z-Cote from BASF); and coated zinc oxides in which coating is added (commercially available as SIH-5 Z-Cote XP-M52 from BASF or Zano 10+ from Umicore). The foregoing SIH-5 Z-Cote XP-M52 is a formulation of zinc oxide/silica/dimethicone methicone copolymer. Also of interest for use with zinc oxide or in addition to any of the foregoing zinc oxides is C₁₂₋₁₅ alkyl benzoate (available as FINSOLV TN) and caprylic/capric triglyceride (available as DERMOL M5). It should be understood that isostearic acid, triglycerides, and isostearates are derived from fatty acids.

A second component present in the synergistic triplet combination according to the present invention is styrene/acrylate copolymer. The styrene/acrylate copolymer is present in a composition according to the present invention in an amount of about 1% to about 10%, preferably about 2% to about 8%, and more preferably about 4% to about 6%.

In a preferred embodiment of the present invention, the styrene/acrylate copolymer is a hollow styrene/acrylate copolymer sphere, which is commercially available as SUNSPHERES LCG SPF BOOSTER from Rohm and Haas of Philadelphia, Pennsylvania. These styrene/acrylate copolymer spheres are manufactured via an emulsion polymerization process and are suitably compatible with all of the other components of the sunscreen compositions described herein. The present invention is not limited to styrene/acrylate copolymer spheres, however, as other materials may be used as substitutes or in addition to the spheres.

The third component present in the synergistic triplet combination according to the present invention is glycol. The glycol for use in the present invention may include, but is not limited to, one or more selected from the group consisting of pentylene glycol (1,2-pentanediol), neopentyl glycol (neopentanediol), caprylyl glycol (1,2-octanediol), ethoxydiglycol, butylene glycol monopropionate, diethylene glycol monobutyl ether, PEG-7 methyl ether, octacosanyl glycol, arachidyl glycol, benzyl glycol, cetyl glycol (1,2-hexanediol), C₁₄₋₁₈ glycol, C₁₅₋₁₈ glycol, lauryl glycol (1,2-dodecanediol), butoxydiglycol, 1,10-decanediol, ethyl hexanediol, or any combinations thereof.

In a preferred embodiment, the glycol that may be included in the synergistic triplet combination is caprylyl glycol (1,2-octanediol), which, in addition to functioning as an optimizing agent, also exhibits antimicrobial activity. For human topical use, a photoprotective composition as described herein may comprise glycol in an amount corresponding to about 0.1 % to about 10% (e.g., to 11%), optionally about 0.25% to about 5% or about 0.5% to about 1.25%, by weight of the photoprotective composition.

The present invention is not limited to the use of glycol, however, as diethylhexyl 2,6-naphthalate (available under the trade name CORAPAN TQ from Symrise, Inc. of Teterboro, New Jersey) may be used in the same amounts as an alternative to or in addition to the glycol.

In the zinc oxide the lattice structure is "reduced." It is believed that in a zinc oxide in which the lattice structure is reduced, the zinc oxide particles possess an excess of zinc ions within an absorbing core. These are localized states and as such may exist within the band gap. However, the electrons and holes may then relax to the excess zinc ion states. Thus, the electrons and holes may be trapped so that they cannot migrate to the surface of the particles and react with absorbed species. The electrons and holes may then recombine at the ionic zinc states accompanied by the release of a photon with an energy equivalent to the difference in the energy levels.

Additionally, a crystal of the zinc oxide is smaller than the crystal size of other zinc oxides, thus giving the zinc oxide more surface area than other zinc oxides. Also, with regard to the zinc oxide of the present invention, smaller crystals are irreversibly agglomerated so that the resulting particles have micron sizes instead of nanometer sizes before and after formulation.

The zinc oxide formed by the combination of the reduced zinc oxide crystals agglomerated with the zinc oxide crystals of larger size may result in the formation of "trap sites" that affect conductivity of the zinc oxide material and therefore the pyroelectric effect. These trap sites, which minimize migration of the electron/hole pairs, may be luminescence trap sites and/or killer sites. Luminescence trap sites and killer sites are foreign ions designed to trap the electrons and positively charged holes and therefore inhibit migration of the electron/hole pairs.

Furthermore, the zinc oxides employed in the present invention are n-doped, thereby facilitating the movement of the electrons in one direction so they eventually cannot move anymore, and thereby resulting in a decrease in conductivity.

The addition of 2.5% of the zinc oxide to replace 5% homosalate in the photoprotective compositions of the present invention, in combination with the styrene/acrylates copolymer and glycol, provides a synergistic effect with regard to efficacy of the photoprotective composition. In particular, an unexpected boost in the in-vitro SPF results is realized, as can be seen below in the Examples. This unexpected boost is present irrespective of the source of the zinc oxide and whether the zinc oxide is coated or uncoated. The unexpected SPF boost is the result of the synergistic combination of the hollow styrene/acrylate copolymer spheres with zinc oxide and caprylyl glycol.

In addition to the above-referenced components, the photoprotective compositions according to the present invention may also include one or more sunscreen agents selected from the group consisting of p-aminobenzoic acid, p-aminobenzoic acid salts, p-aminobenzoic acid derivatives, anthranilates, salicylates, glyceryl ester, dipropyleneglycol esters, cinnamic acid derivatives, dihydroxycinnamic acid derivatives, camphor, camphor derivatives, trihydroxycinnamic acid derivatives, hydrocarbons, dibenzalacetone, benzalacetophenone, naptholsulfonates, dihydroxy-naphthoic acid, dihydroxy-naphthoic acid salts; o-hydroxydiphenyldisulfonates, p-hydroxydiphenyldisulfonates, coumarin derivatives, diazoles, quinine salts, quinoline derivatives, hydroxy- substituted benzophenones, methoxy-substituted benzophenones, uric acids, vilouric acids, tannic acid, tannic acid derivatives, hydroquinone, benzophenones, avobenzone, 4-isopropyldibenzoylmethane, butylmethoxydibenzoylmethane, octocrylene, 4-isopropyl-dibenzoylmethane, metal oxides, titanium dioxide, or any combinations thereof. The one or more sunscreen agents may be present in a photoprotective composition according to the present invention in an amount about 1 wt.% to about 40 wt.%, based on the total weight of the composition.

In addition to the above-referenced components, a photoprotective composition according to the present invention may also include one or more components selected from the group consisting of solvent, water, emulsifier, thickening agent, emollient, moisturizer, humectant, film former/waterproofing agent, bio-active, pH adjuster/chelating agent, preservative, fragrance, effect pigment, color additive, lubricant, elastomer, or any combinations thereof.

The compositions as described herein for testing purposes, particularly with regard to the Examples, were formulated in a laboratory setting. In formulating one exemplary composition, sodium lauroyl sarcosinate is added to water in a first vessel, mixed, and heated. Xanathan gum and glycerin are blended and added to the mixed, heated aqueous sodium lauroyl sarcosinate at a suitable temperature and further heated. In a second vessel, the homosalate, styrene/acrylate copolymer spheres, zinc oxide, and caprylyl glycol (and/or diethylhexyl 2,6-naphthalate) are then combined, mixed, and heated until all components are dissolved. The octisalate is then added and mixed, followed by the octocrylene, benzophenone-3, avobenzone, and other ingredients (e.g., emollients, humectants, film forming agents, primary and secondary emulsifying agents, and the like), and the mixture is heated. The mixture in the first vessel is then homogenized to facilitate uniformity. While being homogenized, the mixture in the second vessel is added and the homogenization is continued for a suitable period of time before being cooled.

In evaluating the pyroelectric effect of zinc oxide, it was believed that if the photons of sunlight could be absorbed by the zinc oxide, then the triplet combination according to the present invention would be more effective at or capable of absorbing photons of light than zinc oxide alone. Because the pyroelectric effect is related to conductivity, it could be measured indirectly by subjecting the zinc oxide to microwaves. Increases in temperature could be used as indicators of pyroelectric effects. Higher temperatures indicate increased conductivity, and therefore increased pyroelectricity. It was determined that the coating on the coated zinc oxide interfered with the conductivity of the zinc oxide.

### Example 1 - comparison of formulations based on uncoated zinc oxides

Several variations of photoprotective compositions were formulated and tested with regard to effectiveness. These formulations incorporated zinc oxide that was uncoated. Samples A-G incorporated uncoated zinc oxide (commercially available from Umicore), and Sample J incorporated an uncoated zinc oxide (commercially available from BASF). The data for Samples A-G and J are presented in Table 1 below. All values are in weight percentages.

**Table 1 - uncoated zinc oxide formulations**

| | Sample A | Sample B | Sample C | Sample D | Sample E | Sample F | Sample G | Sample J |
|---|---|---|---|---|---|---|---|---|
| Homosalate | 10.00 | 10.00 | 10.00 | 10.00 | 10.00 | 10.00 | 10.00 | 10.00 |
| Styrene/acrylate copolymer | -- | 5.00 | 5.00 | 5.00 | 5.00 | -- | -- | 5.00 |
| Zinc oxide (uncoated) | -- | -- | 3.00 | 3.00 | 2.50 | 3.00 | -- | 2.50 |
| Caprylyl glycol | -- | - | -- | 1.00 | 1.00 | -- | 1.00 | 1.00 |
| Octisalate | 5.00 | 5.00 | 5.00 | 5.00 | 5.00 | 5.00 | 5.00 | 5.00 |
| Octocrylene | 2.40 | 2.40 | 2.40 | 2.40 | 2.40 | 2.40 | 2.40 | 2.40 |
| Benzophenone-3 | 6.00 | 6.00 | 6.00 | 6.00 | 6.00 | 6.00 | 6.00 | 6.00 |
| Avobenzone | 3.00 | 3.00 | 3.00 | 3.00 | 3.00 | 3.00 | 3.00 | 3.00 |
| Other | Q.S. to 100 wt.% | | | | | | | |
| | | | | | | | | |
| SPF | 29 | 46 | 109 | 143 | 124 | 74 | 26 | 132 |
| Critical Wavelength (nm) | 377 | 378 | 381 | 381 | 381 | 380 | 377 | 381 |
| UVA/UVB Ratio | 0.743 | 0.748 | 0.782 | 0.796 | 0.786 | 0.785 | 0.742 | 0.793 |

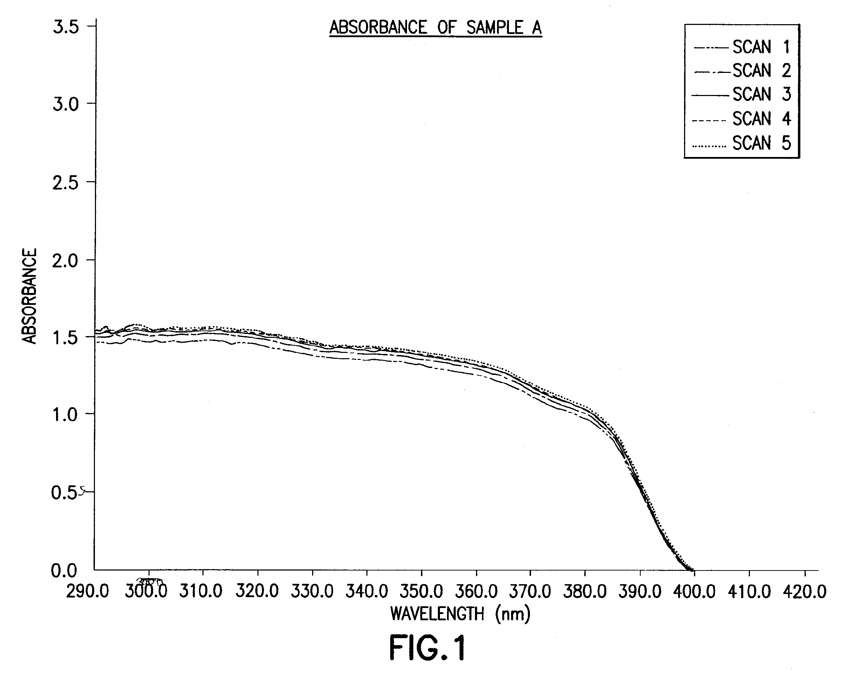

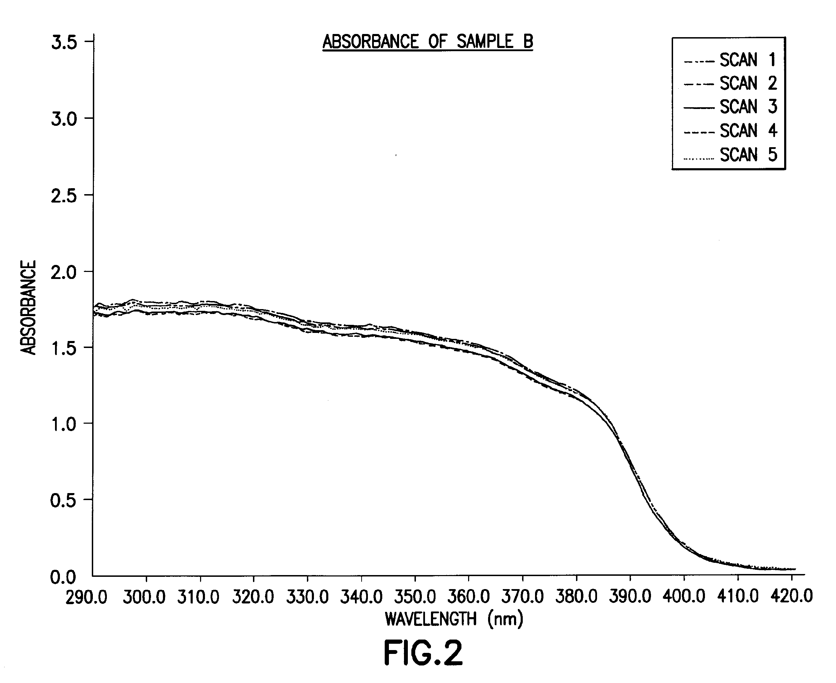

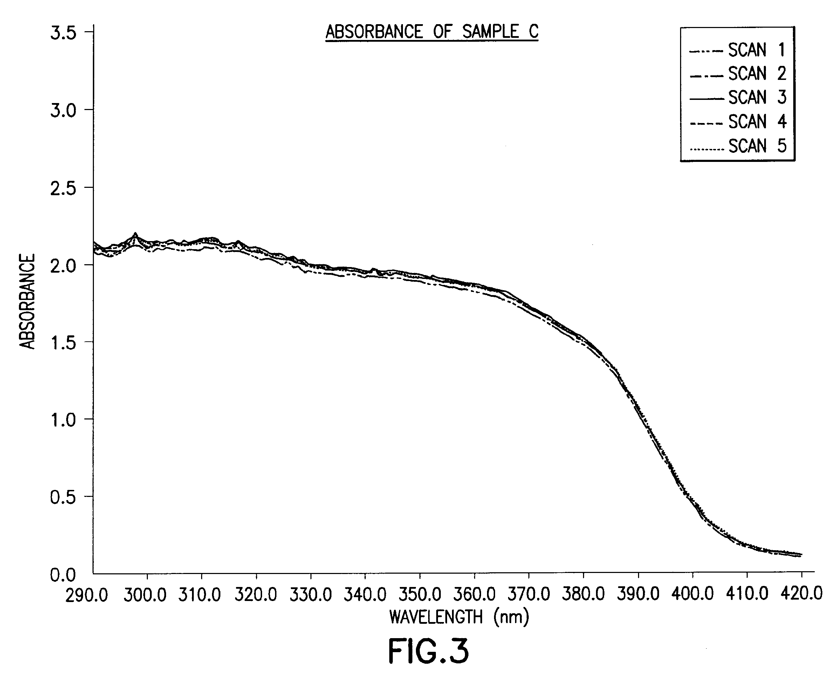

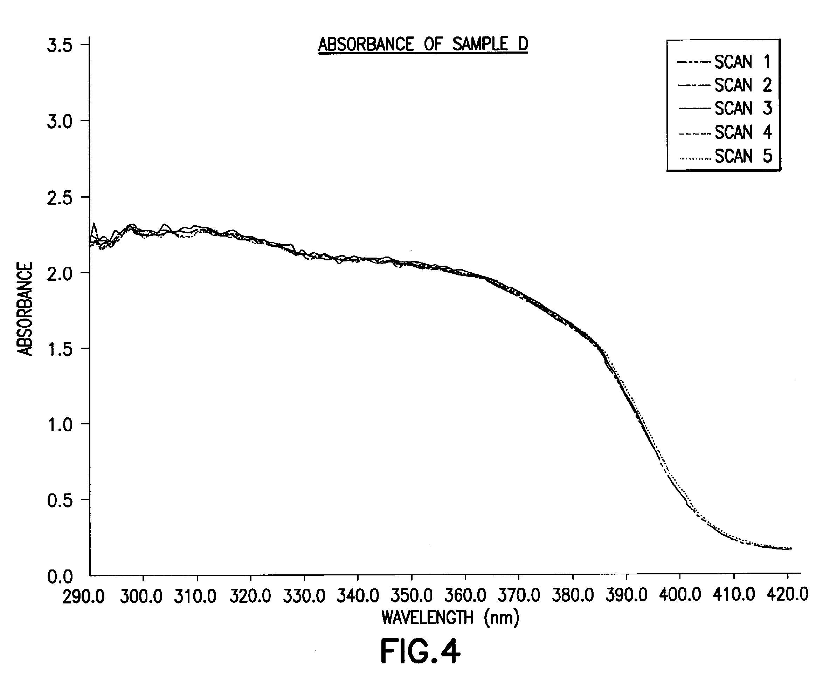

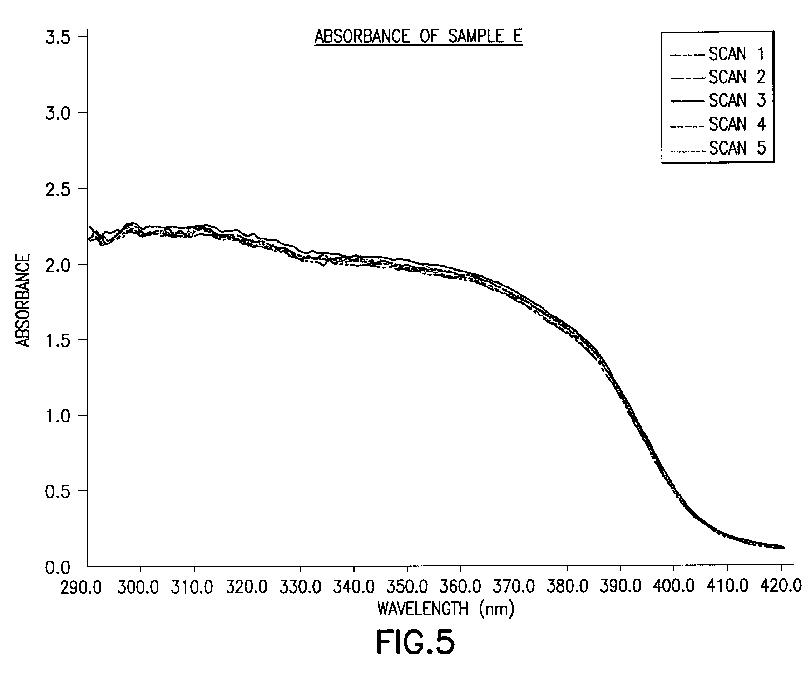

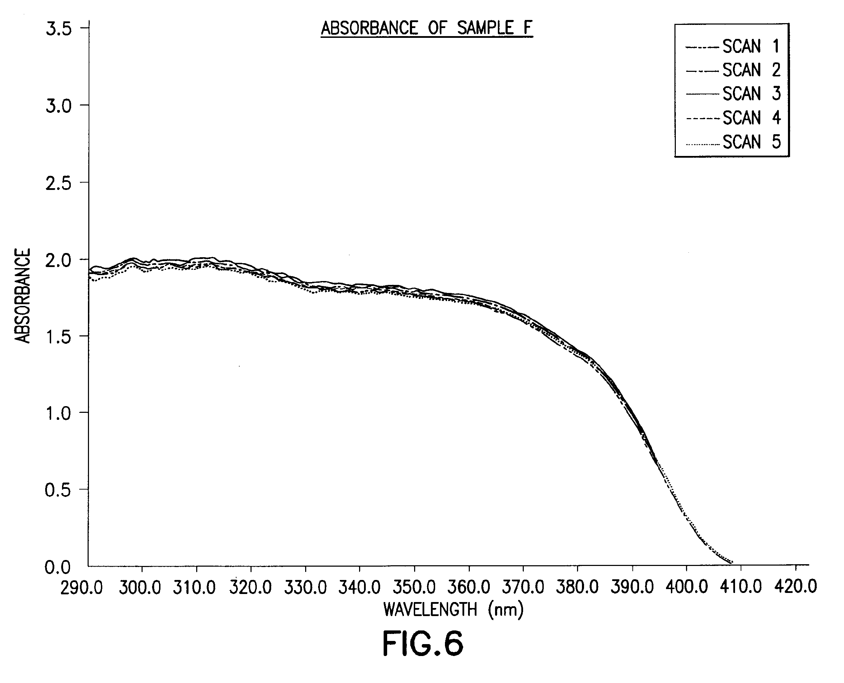

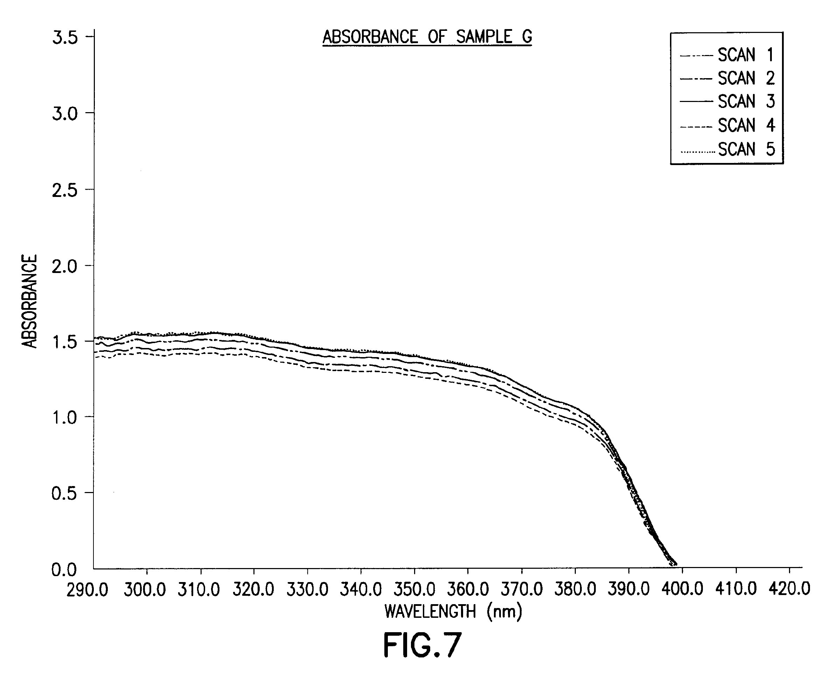

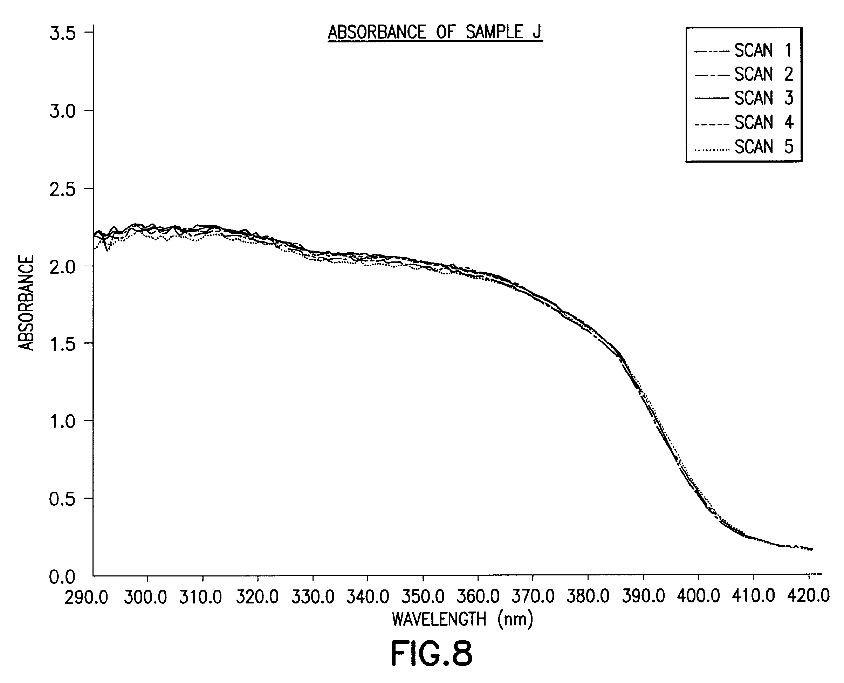

As can be seen in the SPF results for at least Samples D and E, a marked increase in sun protection factor is realized as compared to the other Samples. An increase of similar magnitude is seen with regard to Sample J. Each of the SPF results is an average of five scans made at the indicated wavelength. While not wishing to be bound by any particular theory, it is surmised that the combination of the caprylyl glycol, the zinc oxide, and the styrene/acrylate copolymer spheres causes the SPF to increase more than would any of these components individually. Referring now to FIGS. 1-8, each of the Samples in Table 1 was analyzed for photostability. The absorbance of each Sample was measured using the Labsphere UV 2000S Ultraviolet Transmittance Analyzer (available from Labsphere of North Sutton, NH) ("hereinafter "Labsphere"). In this Example (and in all following Examples) the UVA/UVB ratio was calculated using the Labsphere.

### Example 2 - formulations based on coated zinc oxide

Two photoprotective compositions incorporated zinc oxide that was coated. Sample K (Zano 10+, commercially available from Umicore), whereas Sample L (SIH-5 Z-Cote XP-M52, commercially available from BASF). The formulations incorporating these zinc oxides are shown below in Table 2.

**Table 2 - coated zinc oxide formulations**

| | Sample K | Sample L |
|---|---|---|
| Homosalate | 10.00 | 10.0 |
| Styrene/acrylate copolymer | 5.00 | 5.00 |
| Zinc oxide (coated) | 2.5 | 2.5 |
| Caprylyl glycol | 1.0 | 1.0 |
| Octisalate | 5.00 | 5.00 |
| Octocrylene | 2.40 | 2.40 |
| Benzophenone-3 | 6.00 | 6.00 |
| Avobenzone | 3.00 | 3.00 |
| Other | Q.S. to 100% | |
| | | |
| SPF | 138 | 127 |
| Critical Wavelength (nm) | 381 | 380 |
| UVA/UVB Ratio | 0.792 | 0.777 |

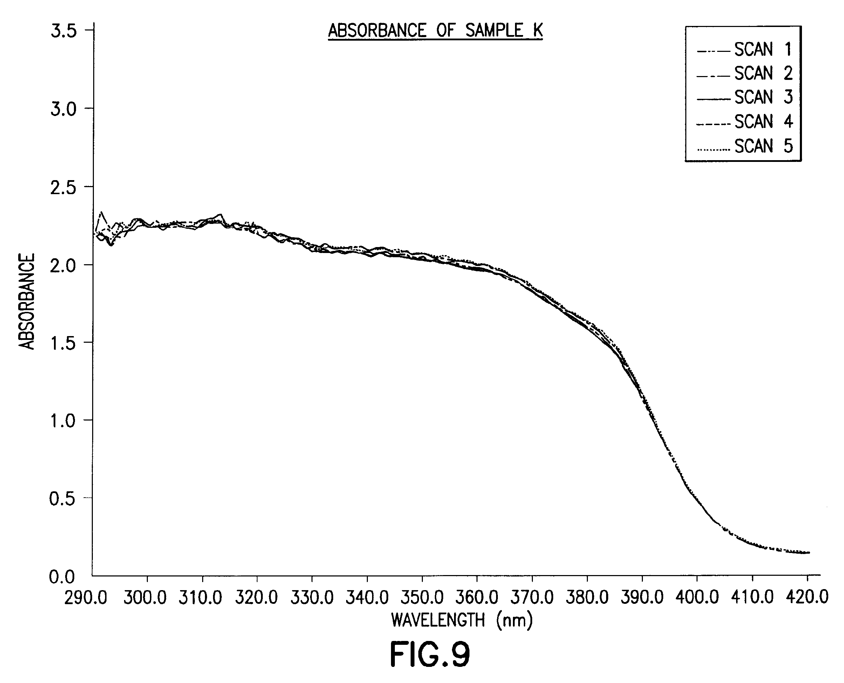

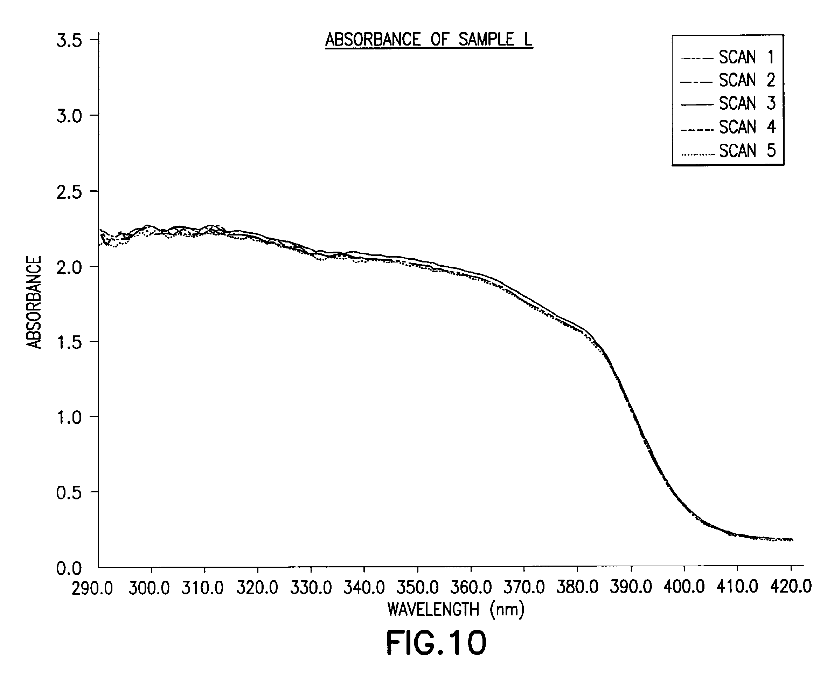

In both Samples, increases in sun protection factor were again realized as compared to Samples A, B, C, F, and G in Example 1. The SPF values are averages of five scans made at the indicated wavelengths. Again, it is surmised that the combination of the caprylyl glycol, the zinc oxide, and the styrene/acrylate copolymer spheres causes the SPF to increase more than would any of these components individually, as indicated by the Samples A, B, C, F, and G in Example 1. Referring now to FIGS. 9 and 10, the absorbance of Samples K and L, respectively, were measured using the Labsphere.

### Example 3 - comparisons of zinc oxide formulations based on dispersions of zinc oxide

Two formulations incorporating dispersions of zinc oxide were analyzed. Sample H was a zinc oxide dispersion comprising 40-60% zinc oxide, 30-59% C₁₂₋₁₅ alkyl benzoate, and 1-5% isostearic acid (ZinClear-IM 50AB from Advanced Nanotechnology), and Sample I was a zinc oxide dispersion comprising 40-60% zinc oxide, 30-59% caprylic/capric triglyceride, and 1-5% glyceryl isostearate (ZinClear-IM 50CCT from Advanced Nanotechnology). The active amount of zinc oxide in either Sample is about 2-3%.

**Table 3 - zinc oxide dispersion formulations**

| | Sample H | Sample I |
|---|---|---|
| Homosalate | 10.00 | 10.00 |
| Styrene/acrylate copolymer | 5.00 | 5.00 |
| Zinc oxide (dispersion) | 5.00 | 5.00 |
| Caprylyl glycol | 1.00 | 1.00 |
| Octisalate | 5.00 | 5.00 |
| Octocrylene | 2.40 | 2.40 |
| Benzophenone-3 | 6.00 | 6.00 |
| Avobenzone | 3.00 | 3.00 |
| Other | Q.S. to 100% | |
| | | |
| SPF | 120 | 119 |
| Critical Wavelength (nm) | 381 | 381 |
| UVA/UVB Ratio | 0.788 | 0.791 |

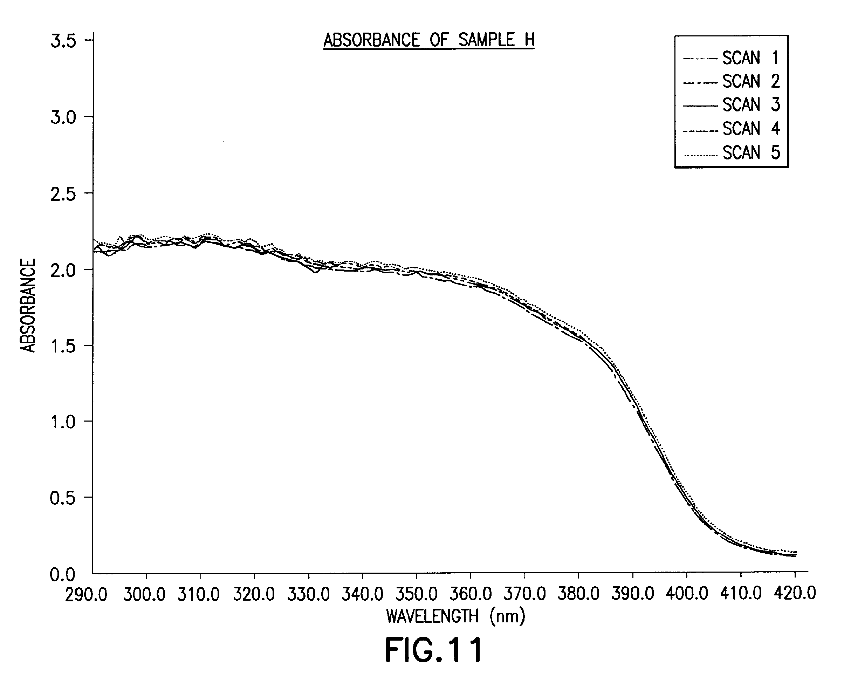

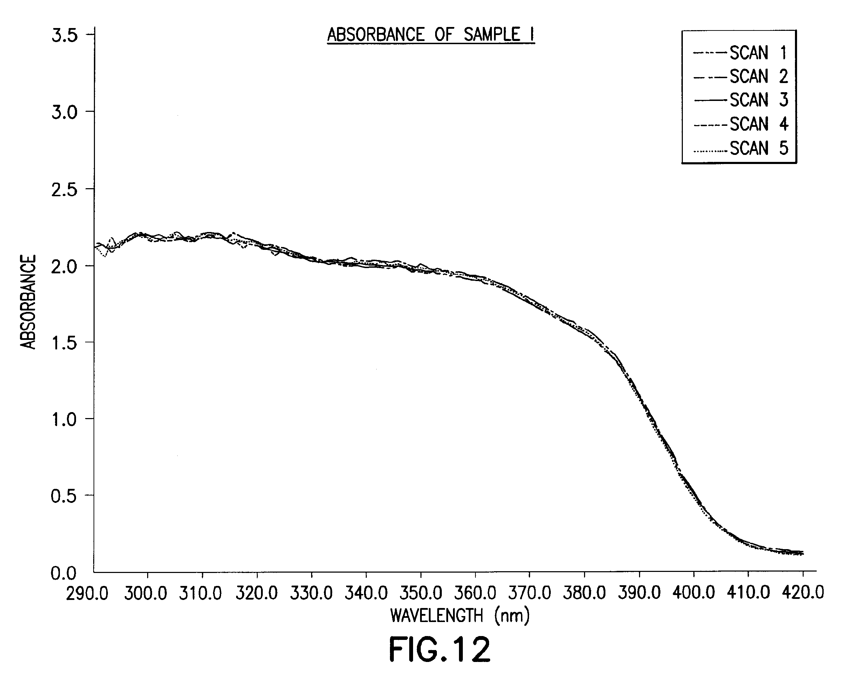

In these samples, SPF values that were comparable to those of Samples D and E were obtained. Referring to FIGS. 11 and 12, the absorbance of Samples H and I, respectively, were measured using the Labsphere. Again without wishing to be bound by any particular theory, it is surmised that zinc oxide in the form of a dispersion provides results that are comparable to those of uncoated zinc oxide.

### Example 4 - comparison of formulations of uncoated zinc oxide with substitution for caprylyl glycol

In one formulation, the caprylyl glycol was replaced with diethylhexyl 2,6-naphthalate. A comparison between this formulation and the formulation of Sample D is shown below with regard to Table 4.

**Table 4 - formulation in which caprylyl glycol was replaced with diethylhexyl 2,6-naphthalate**

| | Sample D | Sample M |
|---|---|---|
| Homosalate | 10.00 | 10.00 |
| Styrene/acrylate copolymer | 5.00 | 5.00 |
| Zinc oxide (uncoated) | 3.00 | 3.00 |
| | 1.00 caprylyl glycol | 1.00 diethylhexyl 2,6-naphthalate |
| Octisalate | 5.00 | 5.00 |
| Octocrylene | 2.40 | 2.40 |
| Benzophenone-3 | 6.00 | 6.00 |
| Avobenzone | 3.00 | 3.00 |
| Other | Q.S. to 100% | |
| | | |
| SPF | 143 | 130 |
| Critical Wavelength (nm) | 381 | 381 |
| UVA/UVB Ratio | 0.796 | 0.794 |

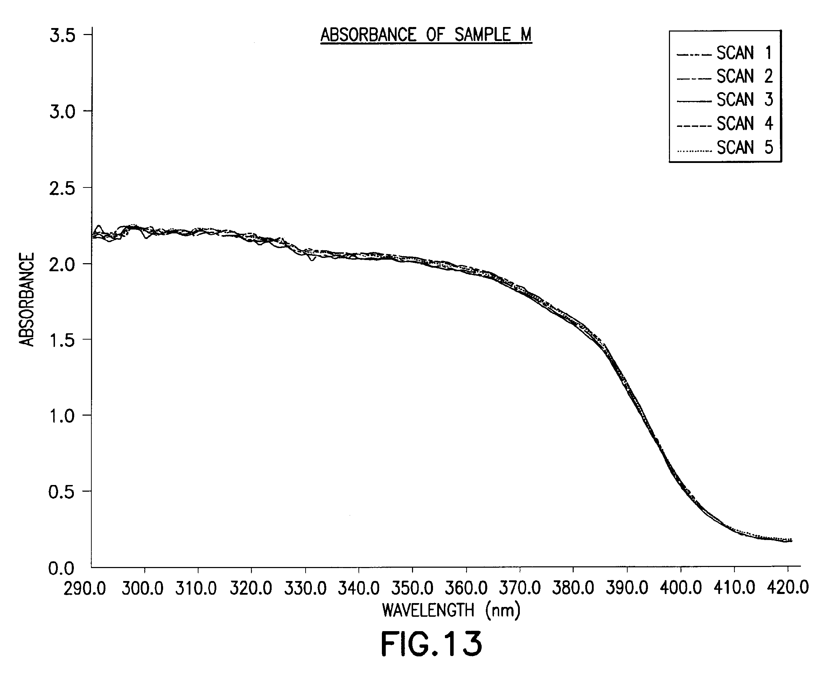

Based on the foregoing, it can be seen that substituting diethylhexyl 2,6-naphthalate for caprylyl glycol provides similar results with regard to SPF. Referring to FIG. 13, the absorbance of Sample M was measured using the Labsphere.

Although this invention has been shown and described with respect to the detailed embodiments thereof, it will be understood by those of skill in the art that various changes may be made and equivalents may be substituted for elements thereof without departing from the scope of the invention. In addition, modifications may be made to adapt a particular situation or material to the teachings of the invention without departing from the essential scope thereof. Therefore, it is intended that the invention not be limited to the particular embodiments disclosed in the above detailed description, but that the invention will include all embodiments falling within the scope of the following claims.

## Claims

1. A photoprotective composition, comprising:
a synergistic triplet combination, comprising,
zinc oxide,
styrene/acrylate copolymer; and
diethylhexyl 2,6 naphthalate,
wherein said synergistic triplet combination provides a boost in a SPF in said photoprotective composition compared to a composition without said triplet combination.

2. The composition of claim 1, wherein said zinc oxide is coated, uncoated, in a dispersion, or any combinations thereof.

3. The composition of claim 1, wherein said zinc oxide is present in an amount of about 1 wt.% to about 15 wt.%, based on a total weight of the composition.

4. The composition of claim 1, wherein said zinc oxide is present in an amount of about 1 wt.% to about 5 wt.%, based on a total weight of the composition.

5. The composition of claim 1, wherein said styrene/acrylate copolymer is a styrene/acrylate copolymer sphere.

6. The composition of claim 1, wherein said styrene/acrylate copolymer is present in an amount about 1 wt.% to about 10 wt.%, based on a total weight of said composition.

7. The composition of claim 1, wherein said styrene/acrylate copolymer is present in an amount about 2 wt.% to about 8 wt.%, based on a total weight of said composition.

8. The composition of claim 1, wherein said diethylhexyl 2,6 naphthalate is present in an amount about 0.25 wt.% to about 5 wt.%, based on a total weight of the composition.

9. The composition of claim 1, further comprising one or more sunscreen agents selected from the group consisting of p-aminobenzoic acid, p-aminobenzoic acid salts, p-aminobenzoic acid derivatives, anthranilates, salicylates, glyceryl ester, dipropyleneglycol esters, cinnamic acid derivatives, dihydroxycinnamic acid derivatives, camphor, camphor derivatives, trihydroxycinnamic acid derivatives, hydrocarbons, dibenzalacetone, benzalacetophenone, naptholsulfonates, dihydroxy-naphthoic acid, dihydroxy-naphthoic acid salts; o- hydroxydiphenyldisulfonates, p-hydroxydiphenyldisulfonates, coumarin derivatives, diazoles, quinine salts, quinoline derivatives, hydroxy- substituted benzophenones, methoxy-substituted benzophenones, uric acids, vilouric acids, tannic acid, tannic acid derivatives, hydroquinone, benzophenones, avobenzone, 4-isopropyldibenzoylmethane, butylmethoxydibenzoylmethane, octocrylene, 4-isopropyl-dibenzoylmethane, metal oxides, titanium dioxide, and any combinations thereof.

10. The photoprotective composition of claim 9, wherein said one or more sunscreen agents are present in an amount between about 1 wt.% to about 40 wt.%, based on the total weight of the composition.
